# EUROPÄISCHE PATENTSCHRIFT

(11) **EP 2 456 367 B1**
(45) Veröffentlichungstag und Bekanntmachung des Hinweises auf die Patenterteilung: **17.09.2014**
(21) Anmeldenummer: 10736685.8
(22) Anmeldetag: 21.07.2010
(51) Int. Cl.: A61B 17/122, A61B 17/10

(54) **CLIPTRÄGERVORRICHTUNG**
CARTRIDGE FOR SURGICAL CLIPS
BOITIERT POUR CLIPS CHIRURICAUX

(30) Priorität: 24.07.2009 DE 102009035756
(43) Veröffentlichungstag der Anmeldung: 30.05.2012
(73) Patentinhaber: Aesculap AG, 78532 Tuttlingen (DE)
(72) Erfinder: DISCH, Alexander, 79104 Freiburg (DE)
(74) Vertreter: Hoeger, Stellrecht & Partner Patentanwälte
(86) Internationale Anmeldenummer: PCT/EP2010/060589
(87) Internationale Veröffentlichungsnummer: WO 2011/009900

(56) Entgegenhaltungen:
- DE-C1- 19 903 752
- DE-U1-202008 004 929
- US-B1- 6 273 253

## Beschreibung

Die vorliegende Erfindung betrifft eine medizinische oder chirurgische Clipträgervorrichtung, welche mindestens eine Halteeinrichtung zum Halten mindestens eines medizinischen oder chirurgischen Clips umfasst. Die mindestens eine Halteeinrichtung umfasst dabei mindestens zwei relativ zueinander bewegliche Halteflächenbereiche zum kraft- und/oder formschlüssigen Halten des mindestens einen Clips und mindestens eine Rückstelleinrichtung zum Bewegen der mindestens zwei Halteflächenbereiche relativ zueinander. Mittels der mindestens einen Rückstelleinrichtung sind die mindestens zwei Halteflächenbereiche nach einer Auslenkung der mindestens zwei Halteflächenbereiche relativ zueinander aus einer Ausgangsstellung in eine ausgelenkte Stellung von der ausgelenkten Stellung in eine kraftneutrale Stellung bringbar, in welcher die mindestens eine Rückstelleinrichtung die mindestens zwei Halteflächenbereiche in einer vorgegebenen Stellung relativ zueinander hält.

Derartige Clipträgervorrichtungen dienen insbesondere zur Aufbewahrung eines Vorrats an medizinischen oder chirurgischen Clips (auch "Ligaturclips", "Ligaturklammern", "(hämostatische) Klammern" oder "Clips" genannt) und sind aus dem Stand der Technik bekannt.

Bei den aus dem Stand der Technik bekannten Clipträgervorrichtungen werden die Clips beispielsweise mittels einer reibschlüssigen Verbindung an elastischen Klemmvorrichtungen der Clipträgervorrichtung gehalten, sodass beim Entnehmen eines Clips von der Clipträgervorrichtung die Reibwirkung der jeweiligen Klemmvorrichtung einen Materialabrieb von der Klemmvorrichtung und somit eine Verunreinigung des Clips und/oder eine Beschädigung des Clips bewirkt. Insbesondere dann, wenn die elastischen Klemmvorrichtungen an Außenseiten der Clips angreifen, kann ferner bei den aus dem Stand der Technik bekannten Clipträgervorrichtungen erforderlich sein, dass die elastischen Klemmvorrichtungen während des gesamten Entnahmeprozesses eines Clips mittels einer den Clip aufnehmenden Applikatorzange weg gehalten werden müssen, sodass eine einfache und sichere Clipentnahme durch die elastischen Klemmvorrichtungen behindert wird.

Clipträgervorrichtungen sind beispielsweise aus der DE 199 03 752 C1, der US Clipträgervorrichtungen sind beispielsweise aus der DE 199 03 752 C1, der US 6,273,253 B1 und der DE 20 2008 004 929 U1 bekannt.

Insbesondere die Patentschrift DE 199 03 752 C1 offenbart den Gegenstand der Präambel des Anspruchs 1.

Der vorliegenden Erfindung liegt die Aufgabe zugrunde, eine Clipträgervorrichtung bereitzustellen, welche eine besonders einfache Entnahme eines Clips von der Clipträgervorrichtung ermöglicht.

Diese Aufgabe wird erfindungsgemäßlaut Anspruch 1 dadurch gelöst, dass die mindestens eine Halteeinrichtung mindestens eine der mindestens einen Rückstelleinrichtung entgegenwirkende Dämpfungseinrichtung zur Dämpfung einer Bewegung der mindestens zwei Halteflächenbereiche relativ zueinander von der ausgelenkten Stellung in Richtung der Ausgangsstellung zurück umfasst.

Dadurch, dass die mindestens eine Halteeinrichtung mindestens eine Dämpfungseinrichtung umfasst, welche einer Wirkung der mindestens einen Rückstelleinrichtung, das heißt einer Zurückbewegung der mindestens zwei Halteflächenbereiche nach einer Auslenkung derselben, entgegenwirkt, wird die Bewegung der mindestens zwei Halteflächenbereiche relativ zueinander nach einer Auslenkung zurück in Richtung der Ausgangsstellung verlangsamt, sodass eine Relaxationszeit, welche nach der Auslenkung der mindestens zwei Halteflächenbereiche vergeht, bis die mindestens zwei Halteflächenbereiche wieder eine kraftneutrale Stellung eingenommen haben, in welcher die mindestens eine Rückstelleinrichtung keine weitere Bewegung der mindestens zwei Halteflächenbereiche bewirkt, vergrößert wird. Die mindestens eine Dämpfungseinrichtung ist dabei vorzugsweise so ausgebildet, dass die Relaxationszeit der mindestens zwei Halteflächenbereiche größer, vorzugsweise erheblich, beispielsweise um eine Größenordnung, größer, ist als eine Entnahmezeit, welche benötigt wird, um mindestens einen Clip von der mindestens einen Clipträgervorrichtung zu entnehmen.

Ferner ist dadurch, dass die mindestens eine Halteeinrichtung mindestens eine der mindestens einen Rückstelleinrichtung entgegenwirkende Dämpfungseinrichtung zur Dämpfung einer Bewegung der mindestens zwei Halteflächenbereiche relativ zueinander von der ausgelenkten Stellung in Richtung der Ausgangsstellung zurück umfasst, insbesondere auch eine Reduktion der mittels der mindestens einen Rückstelleinrichtung und der mindestens zwei Halteflächenbereiche auf einen an der Clipträgervorrichtung gehaltenen Clip wirkenden Kraft und damit eine Reduktion der den Clip haltenden Reibwirkung beim Entnehmen des Clips gewährleistet. Dies hat zum Vorteil, dass bei der Entnahme des mindestens einen Clips ein Materialabrieb von der Clipträgervorrichtung minimiert wird. Eine unerwünschte Verunreinigung des Clips wird somit ebenfalls verringert, insbesondere ganz vermieden.

Die Ausgangsstellung, in welcher die mindestens zwei Halteflächenbereiche vor einer Auslenkung derselben angeordnet sind, ist vorzugsweise eine kraftneutrale Stellung, in welcher eine mittels der mindestens einen Rückstelleinrichtung auf die mindestens zwei Halteflächenbereiche ausgeübte Kraft durch eine dieser Kraft entgegenwirkende Kraft kompensiert wird. Insbesondere kann hierbei vorgesehen sein, dass diese der mindestens einen Rückstelleinrichtung entgegenwirkende Kraft von einem Anschlag für die mindestens zwei Halteflächenbereiche und/oder einem an der Clipträgervorrichtung angeordneten Clip aufgebracht wird.

Bei einer Weiterbildung der Erfindung ist vorgesehen, dass die Clipträgervorrichtung einen Grundkörper umfasst. Mindestens ein Halteflächenbereich ist vorzugsweise relativ zu dem Grundkörper beweglich ausgebildet. Einerseits kann hierbei vorgesehen sein, dass einer der mindestens zwei Halteflächenbereiche fest an dem Grundkörper angeordnet ist und der andere der mindestens zwei Halteflächenbereiche beweglich an dem Grundkörper angeordnet ist. Vorzugsweise sind jedoch die mindestens zwei Halteflächenbereiche relativ zu dem Grundkörper beweglich ausgebildet. Auf diese Weise kann ein an der Clipträgervorrichtung angeordneter Clip besonders einfach und flexibel mittels der mindestens zwei Halteflächenbereiche gehalten werden.

Eine besonders einfache Anordnung des mindestens einen Clips an der Clipträgervorrichtung ist insbesondere dann möglich, wenn die mindestens eine Halteeinrichtung eine Auflagefläche für den mindestens einen Clip umfasst. Auf diese Weise ist insbesondere eine einfache Zentrierung des mindestens einen Clips an der mindestens einen Halteeinrichtung der Clipträgervorrichtung möglich.

Günstig ist es, wenn mindestens ein Halteflächenbereich der mindestens einen Halteeinrichtung Teil der Auflagefläche für den mindestens einen Clip ist. Insbesondere dann, wenn einer der mindestens zwei Halteflächenbereiche fest mit dem Grundkörper der Clipträgervorrichtung verbunden ist und lediglich der andere der mindestens zwei Halteflächenbereiche relativ zu dem Grundkörper beweglich ausgebildet ist, kann so vorgesehen sein, dass der fest an dem Grundkörper angeordnete Halteflächenbereich einen Teil der Auflagefläche für den mindestens einen Clip bildet.

Vorteilhaft kann es sein, wenn mindestens ein Halteflächenbereich quer zu der Auflage für den mindestens einen Clip, insbesondere im Wesentlichen senkrecht zu der Auflagefläche für den mindestens einen Clip, ausgerichtet ist. Auf diese Weise ist ein besonders einfaches Anlegen des mindestens einen Clips zum Anordnen desselben an der Clipträgervorrichtung möglich.

Bei einer Ausgestaltung der Erfindung kann vorgesehen sein, dass mindestens ein Halteflächenbereich gegenüberliegend von der Auflagefläche und insbesondere der Auflagefläche zugewandt angeordnet ist. Auf diese Weise kann der mindestens eine an der Clipträgervorrichtung anzuordnende Clip beispielsweise dadurch besonders einfach an der Clipträgervorrichtung gehalten werden, dass der mindestens eine Clip zwischen die Auflagefläche und den der Auflagefläche gegenüberliegenden mindestens einen Halteflächenbereich geklemmt wird.

Günstig ist es, wenn mindestens ein Halteflächenbereich in Richtung der Auflagefläche beweglich ausgebildet ist. Auf diese Weise kann beispielsweise ein zumindest teilweise zwischen dem mindestens einen Halteflächenbereich und der Auflagefläche angeordneter Clip besonders einfach an der Clipträgervorrichtung eingespannt werden.

Alternativ oder ergänzend hierzu kann vorgesehen sein, dass mindestens ein Halteflächenbereich von der Auflagefläche weg beweglich ausgebildet ist. So kann insbesondere vorgesehen sein, dass der mindestens eine Halteflächenbereich von der Auflagefläche weg bewegt wird, um mindestens einen Clip zwischen den mindestens einen Halteflächenbereich und die Auflagefläche einzuspannen.

Ferner kann vorgesehen sein, dass mindestens ein Halteflächenbereich parallel zu der Auflagefläche beweglich ausgebildet ist. Dies ist insbesondere dann von Vorteil, wenn die Funktion des kraft- und/oder formschlüssigen Haltens des mindestens einen Clips mittels der mindestens zwei relativ zueinander beweglichen Halteflächenbereiche getrennt von der Funktion der Auflage des mindestens einen Clips auf der mindestens einen Auflagefläche realisiert werden soll.

Die Clipträgervorrichtung umfasst vorzugsweise mindestens ein Auflageelement, dessen Oberfläche zumindest einen Teil der Auflagefläche für den mindestens einen Clip bildet. Das Auflageelement kann beispielsweise als ein Teil des Grundkörpers der Clipträgervorrichtung ausgebildet oder fest oder beweglich mit dem Grundkörper der Clipträgervorrichtung verbunden sein.

Günstig ist es, wenn das mindestens eine Auflageelement zumindest teilweise aus einem elastischen Material gebildet ist. Auf diese Weise ist insbesondere eine Deformation des mindestens einen Auflageelements bei der Anordnung und/oder Entnahme mindestens eines Clips an beziehungsweise von der Clipträgervorrichtung möglich, sodass sowohl bei der Anordnung als auch bei der Entnahme Fertigungs- und/oder Handhabungsungenauigkeiten ausgeglichen werden können.

Vorteilhaft ist es, wenn das mindestens eine Auflageelement eine Deckschicht umfasst, deren Oberfläche zumindest einen Teil der Auflagefläche für den mindestens einen Clip bildet. Durch die Verwendung einer Deckschicht für das mindestens eine Auflageelement kann gewährleistet werden, dass ein Grundmaterial des mindestens einen Auflageelements nicht in direkten Kontakt mit dem mindestens einen an der Clipträgervorrichtung anzuordnenden Clip kommt. Eine Verunreinigung des mindestens einen Clips mittels des Grundmaterials des mindestens einen Auflageelements ist durch die Verwendung einer Deckschicht auf dem mindestens einen Auflageelement somit wirksam verhindert.

Die mindestens zwei Halteflächenbereiche der mindestens einen Halteeinrichtung sind vorzugsweise einander zugewandt angeordnet. Auf diese Weise kann insbesondere ein zwischen den mindestens zwei Halteflächenbereichen angeordneter Clip besonderes einfach an der Clipträgervorrichtung angeordnet werden.

Alternativ hierzu kann vorgesehen sein, dass die mindestens zwei Halteflächenbereiche einander abgewandt angeordnet sind. Auf diese Weise ist insbesondere ein Clip, welcher die mindestens zwei Halteflächenbereiche umgreift, an der Clipträgervorrichtung anordenbar. Die mindestens zwei Halteflächenbereiche greifen dabei vorzugsweise auf einander gegenüberliegenden Innenflächen des mindestens einen an der Clipträgervorrichtung angeordneten Clips an und halten den mindestens einen Clip beispielsweise mittels einer form- und/oder kraftschlüssigen Verbindung.

Die mindestens zwei Halteflächenbereiche sind vorzugsweise ausgehend von der Ausgangsstellung voneinander weg beweglich. Insbesondere dann, wenn mindestens ein Clip zwischen die mindestens zwei Halteflächenbereiche gespannt werden soll, kann dadurch, dass die mindestens zwei Halteflächenbereiche ausgehend von der Ausgangsstellung voneinander weg beweglich sind, der zur Anordnung des mindestens einen Clips benötigte Raum geschaffen werden. Die mindestens zwei Halteflächenbereiche werden dann vorzugsweise mittels der mindestens einen Rückstelleinrichtung auf einander zu bewegt, sodass der zwischen den mindestens zwei Halteflächenbereichen angeordnete mindestens eine Clip zwischen die mindestens zwei Halteflächenbereiche eingespannt wird.

Alternativ oder ergänzend hierzu kann vorgesehen sein, dass die mindestens zwei Halteflächenbereiche ausgehend von der Ausgangsstellung aufeinander zu beweglich sind. Insbesondere dann, wenn der mindestens eine Clip die mindestens zwei Halteflächenbereiche umgreift, sodass die mindestens zwei Halteflächenbereiche in einem Lagerzustand des mindestens einen Clips an Innenseiten des mindestens einen Clips angreifen, kann vorgesehen sein, dass die mindestens zwei Halteflächenbereiche aufeinander zu beweglich sind, um eine form- und/oder kraftschlüssige Verbindung zwischen den mindestens zwei Halteflächenbereichen und dem mindestens einen Clip zu lösen. Die mindestens zwei Halteflächenbereiche werden dabei vorzugsweise dadurch aufeinander zu bewegt, dass der mindestens eine Clip mittels einer Applikatorzange so verformt wird, dass die einander zugewandten Innenseiten des mindestens einen Clips, an welchen die mindestens zwei Halteflächenbereiche angreifen, aufeinander zu bewegt werden.

Die mindestens eine Halteeinrichtung umfasst vorzugsweise mindestens ein Halteelement, dessen Oberfläche zumindest einen Teil mindestens eines Halteflächenbereichs bildet. Das mindestens eine Halteelement ist vorzugsweise zumindest teilweise beweglich an dem Grundkörper der Clipträgervorrichtung angeordnet.

Günstig ist es, wenn das mindestens eine Halteelement zumindest teilweise aus einem elastischen Material gebildet ist. Dabei kann vorgesehen sein, dass das elastische Material zumindest einen Teil der mindestens einen Rückstelleinrichtung der mindestens einen Halteeinrichtung bildet.

Vorteilhaft kann es sein, wenn das mindestens eine Halteelement eine Deckschicht umfasst, deren Oberfläche zumindest einen Teil mindestens eines Halteflächenbereichs bildet. Auf diese Weise kann als Grundmaterial für das mindestens eine Halteelement ein beliebiges Material gewählt werden, ohne auf die für die übliche Verwendung des medizinischen oder chirurgischen Clips notwendigen Reinheitserfordernisse Rücksicht zu nehmen, da die Deckschicht einen direkten Kontakt des mindestens einen an der Clipträgervorrichtung angeordneten Clips mit dem Grundmaterial des mindestens einen Halteelements verhindert, sodass der mindestens eine an der Clipträgervorrichtung angeordnete Clip nicht mit dem Grundmaterial des mindestens einen Halteelements verunreinigt werden kann.

Bei einer Ausgestaltung der Erfindung kann vorgesehen sein, dass die mindestens eine Rückstelleinrichtung und die mindestens eine Dämpfungseinrichtung separate Bauteile der mindestens einen Halteeinrichtung sind.

Alternativ hierzu kann vorgesehen sein, dass die mindestens eine Rückstelleinrichtung einstückig mit der mindestens einen Dämpfungseinrichtung ausgebildet ist.

Günstig ist es, wenn die mindestens eine Rückstelleinrichtung und die mindestens eine Dämpfungseinrichtung aus demselben Material gebildet sind. Auf diese Weise sind die mindestens eine Rückstelleinrichtung und die mindestens eine Dämpfungseinrichtung und damit die gesamte Clipträgervorrichtung besonders einfach herstellbar.

Das Material der mindestens einen Rückstelleinrichtung und/oder der mindestens einen Dämpfungseinrichtung ist vorzugsweise ein viskoelastisches Material. Unter einem viskoelastischen Material ist in dieser Beschreibung und den beigefügten Ansprüchen ein elastisches Material zu verstehen, welches sich nach einer Deformation nur langsam zurückbildet.

Grundsätzlich sind sämtliche Materialien mit ausgeprägtem viskoelastischen Verhalten verwendbar.

Insbesondere kann vorgesehen sein, dass das viskoelastische Material Silikon, Kautschuk, thermoplastische Elastomere (TPE), Polyethylen, Polypropylen und/oder Polyurethan umfasst oder aus Silikon, Kautschuk, thermoplastischen Elastomeren (TPE), Polyethylen, Polypropylen und/oder Polyurethan gebildet ist.

Bei einer Ausgestaltung der Erfindung kann vorgesehen sein, dass das viskoelastische Material einen geschäumten Werkstoff umfasst. Insbesondere können hierzu viskoelastische Schaumstoffe, welche auch als "Slow Recovery" - Schaumstoffe bekannt sind, verwendet werden. Auf diese Weise ist eine besonders einfache Herstellung der Clipträgervorrichtung möglich. Insbesondere kann hierbei vorgesehen sein, dass die Clipträgervorrichtung einstückig aus einem schäumbaren Kunststoff hergestellt und nur partiell aufgeschäumt wird.

Alternativ oder ergänzend hierzu kann vorgesehen sein, dass das viskoelastische Material einen porösen Werkstoff umfasst.

Grundsätzlich kann vorgesehen sein, dass die Clipträgervorrichtung zur Entnahme eines Clips zumindest abschnittsweise plastisch verformt wird, sodass die Clipträgervorrichtung zumindest teilweise nicht mehr in einen Ausgangszustand zurückkehrt.

Günstig ist, wenn das viskoelastische Material ein Material ist, welches nach einer Verformung im Wesentlichen vollständig in einen Ursprungszustand zurückkehrt. Der Ursprungszustand des viskoelastischen Materials ist dabei insbesondere der Zustand, in welchem sich das viskoelastische Material unmittelbar nach seiner Herstellung befindet. Das viskoelastische Material weist dabei vorzugsweise ohne äußere Krafteinwirkung seine maximale Ausdehnung auf. Günstig ist es ferner, wenn das viskoelastische Material eine Relaxationszeit, welche nach einer Deformation des viskoelastischen Materials bis zur zumindest teilweisen Wiederherstellung des Ursprungszustands vergeht, von mindestens ungefähr 0,5 Sekunden aufweist. Dadurch ist gewährleistet, dass die mindestens zwei Halteflächenbereiche nach einer Auslenkung aus einer Ausgangsstellung in eine ausgelenkte Stellung ausreichend langsam in eine kraftneutrale Stellung übergehen, sodass der mindestens eine an der Clipträgervorrichtung gehaltene Clip nach einer Auslenkung der mindestens zwei Halteflächenbereiche von der Clipträgervorrichtung entnommen werden kann, bevor die mindestens zwei Halteflächenbereiche mittels der mindestens einen Rückstelleinrichtung wieder an die Innenseite des Clips angelegt werden und dort eine unerwünschte Reibwirkung erzeugen.

Die Relaxationszeit kann grundsätzlich sehr lang sein, sodass sich das viskoelastische Material zumindest näherungsweise zunächst plastisch verformt.

Alternativ hierzu kann es jedoch auch günstig sein, wenn das viskoelastische Material eine Relaxationszeit von höchstens ungefähr 2 Sekunden aufweist. Insbesondere zur Anordnung mindestens eines Clips an der Clipträgervorrichtung ist dadurch gewährleistet, dass die mindestens zwei Halteflächenbereiche nach ihrer Auslenkung ausreichend zügig mittels der mindestens einen Rückstelleinrichtung in eine kraftneutrale Stellung, insbesondere in die Lagerstellung, in welcher der mindestens eine Clip mittels der mindestens zwei Halteflächenbereiche an der Clipträgervorrichtung gehalten ist, gebracht werden.

Eine besonders einfache Herstellung der Clipträgervorrichtung ist insbesondere dann möglich, wenn die mindestens eine Halteeinrichtung ein viskoelastisches Material umfasst oder aus einem viskoelastischen Material gebildet ist.

Die Clipträgervorrichtung umfasst vorzugsweise mindestens einen Clip oder bildet ein Set oder eine Kombination, welche mindestens eine Clipträgervorrichtung und mindestens einen Clip umfasst.

Günstig ist es, wenn der mindestens eine Clip zwei Schenkel umfasst, welche einen Ursprungswinkel von weniger als 180° miteinander einschließen. Der Ursprungswinkel ist dabei der Winkel, welcher von den zwei Schenkeln des mindestens einen Clips zumindest näherungsweise eingeschlossen wird, wenn der mindestens eine Clip an der Clipträgervorrichtung gehalten ist. Der Ursprungswinkel entspricht dabei vorzugsweise zumindest näherungsweise dem Winkel, den die zwei Schenkel des mindestens einen Clips einschließen, wenn keine Kraft auf den Clip wirkt.

Insbesondere dann, wenn der mindestens eine Clip zwei Schenkel umfasst, welche eine Ursprungswinkel von weniger als 120° miteinander einschließen, kann der mindestens eine Clip besonders einfach dadurch an der Clipträgervorrichtung angeordnet werden, dass der mindestens eine Clip die mindestens zwei Halteflächenbereiche umgreift. Die mindestens zwei Halteflächenbereiche greifen dann vorzugsweise an einander zugewandten Innenseiten der zwei Schenkel des mindestens einen Clips an.

Günstig ist es, wenn der mindestens eine Clip eine Rückstelleinrichtung zum Wiederherstellen des von den zwei. Schenkeln eingeschlossenen Ursprungswinkels nach einer Deformation des mindestens einen Clips umfasst.

Insbesondere kann dabei vorgesehen sein, dass eine Relaxationszeit des mindestens einen Clips, welche nach einer Deformation des mindestens einen Clips bis zur zumindest teilweisen Wiederherstellung des von den zwei Schenkeln eingeschlossenen Ursprungswinkels vergeht, kleiner ist als eine Relaxationszeit der mindestens einen Halteeinrichtung, welche nach einer Auslenkung der mindestens zwei Halteflächenbereiche relativ zueinander bis zur zumindest teilweisen Rückkehr in die Ausgangsstellung vergeht. Auf diese Weise ist insbesondere gewährleistet, dass der mindestens eine Clip von der Clipträgervorrichtung abgehoben werden kann, bevor die mindestens zwei Halteflächenbereiche nach deren Auslenkung in den Ausgangszustand zurückkehren, insbesondere bevor die mindestens zwei Halteflächenbereiche nach deren Auslenkung und dem Lösen der Verbindung zwischen dem mindestens einen Clip und der Clipträgervorrichtung erneut an dem mindestens einen Clip angreifen.

Die mindestens eine Halteeinrichtung weist vorzugsweise eine Form auf, welche zumindest teilweise und zumindest näherungsweise komplementär zu der Form einer Innenseite des mindestens einen Clips ausgebildet ist. Auf diese Weise ist eine besonders feste Verbindung zwischen der Clipträgervorrichtung und dem mindestens einen Clip und somit ein besonders sicherer Halt des mindestens einen Clips an der Clipträgervorrichtung möglich, wenn der mindestens eine Clip zumindest teilweise mit seiner Innenseite an der mindestens einen Halteeinrichtung anliegt.

Vorzugsweise weist mindestens ein Halteflächenbereich eine Form auf, welche zumindest teilweise und zumindest näherungsweise komplementär zu der Form einer Innenseite des mindestens einen Clips ausgebildet ist. Insbesondere dann, wenn der mindestens eine Halteflächenbereich zum Halten des mindestens einen Clips an der Innenseite des mindestens einen Clips angreift, kann auf diese Weise ein besonders sicherer Kraft- und/oder Formschluss zwischen dem mindestens einen Clip und der Clipträgervorrichtung hergestellt werden.

Alternativ oder ergänzend hierzu kann vorgesehen sein, dass mindestens ein Halteflächenbereich eine Form aufweist, welche zumindest teilweise und zumindest näherungsweise komplementär zu einer Außenseite des mindestens einen Clips ausgebildet ist. Insbesondere dann, wenn der mindestens eine Halteflächenbereich zum Halten des mindestens einen Clips an der Außenseite des mindestens einen Clips angreift, ist auf diese Weise ein besonders sicherer Halt des mindestens einen Clips an der Clipträgervorrichtung mittels eines Kraft- und/oder Formschlusses möglich.

Bei einer Ausgestaltung der Erfindung kann vorgesehen sein, dass die Clipträgervorrichtung mindestens eine Kopplungseinrichtung zum Koppeln des mindestens einen Clips mit der mindestens einen Halteeinrichtung umfasst. Insbesondere kann hierbei vorgesehen sein, dass mittels der mindestens einen Kopplungseinrichtung eine formschlüssige Verbindung zwischen dem mindestens einen Clip und der Clipträgervorrichtung herstellbar ist.

Die Kopplungseinrichtung umfasst vorzugsweise mindestens zwei Kopplungselemente, welche in einer Lagerstellung der mindestens einen Halteeinrichtung, in welcher der mindestens eine Clip an der mindestens einen Halteeinrichtung gehalten ist, miteinander in Eingriff bringbar sind. Auf diese Weise ist eine besonders sichere Verbindung zwischen dem mindestens einen Clip und der Clipträgervorrichtung gewährleistet, sodass ein unerwünschtes Abheben des mindestens einen Clips von der Clipträgervorrichtung wirksam verhindert ist.

Günstig ist es, wenn mindestens ein Kopplungselement der Kopplungseinrichtung als ein Vorsprung ausgebildet ist. Insbesondere dann, wenn mindestens ein Kopplungselement der Kopplungseinrichtung als eine Aufnahme ausgebildet ist, ist eine besonders einfache Kopplung des mindestens einen Clips mit der Clipträgervorrichtung möglich.

Mindestens ein Kopplungselement der Kopplungseinrichtung ist vorzugsweise an dem mindestens einen Clip angeordnet.

Alternativ oder ergänzend hierzu kann vorgesehen sein, dass mindestens ein Kopplungselement der Kopplungseinrichtung an der mindestens einen Halteeinrichtung angeordnet ist.

Insbesondere kann vorgesehen sein, dass der Clip und/oder die Clipträgervorrichtung mit kleinen Absätzen und/oder Vorsprüngen versehen sind, sodass eine reib- und/oder formschlüssige Verbindung zwischen dem Clip und der Clipträgervorrichtung besonders einfach herstellbar ist.

Der vorliegenden Erfindung liegt ferner die Aufgabe zugrunde, ein Verfahren zum Entnehmen eines medizinischen oder chirurgischen Clips von einer Halteeinrichtung einer medizinischen oder chirurgischen Clipträgervorrichtung für mindestens einen medizinischen oder chirurgischen Clip bereitzustellen, welches ein besonders einfaches Entnehmen des mindestens einen Clips von der Clipträgervorrichtung ermöglicht.

Bei den bekannten Verfahren werden mindestens zwei Halteflächenbereiche der Halteeinrichtung zum Lösen einer kraft- und/oder formschlüssigen Verbindung zwischen dem Clip und der Halteeinrichtung relativ zueinander ausgelenkt, wobei die mindestens eine Halteeinrichtung mindestens eine Rückstelleinrichtung zum Bewegen der mindestens zwei Halteflächenbereiche relativ zueinander in eine kraftneutrale Stellung, in welcher die mindestens eine Rückstelleinrichtung die mindestens zwei Halteflächenbereiche in einer vorgegebenen Stellung relativ zueinander hält, nach einer Auslenkung der mindestens zwei Halteflächenbereiche relativ zueinander aus einer Ausgangsstellung in eine ausgelenkte Stellung umfasst.

Die vorstehend beschriebene Aufgabe wird bei dem erfindungsgemäßen Verfahren dadurch gelöst, dass die mindestens eine Halteeinrichtung mindestens eine der mindestens einen Rückstelleinrichtung entgegenwirkende Dämpfungseinrichtung zur Dämpfung einer Bewegung der mindestens zwei Halteflächenbereiche relativ zueinander von der ausgelenkten Stellung in Richtung der Ausgangsstellung zurück umfasst und dass der Clip von der Halteeinrichtung entnommen wird, bevor die mindestens zwei Halteflächenbereiche in eine vor der Auslenkung bestehende Ausgangsstellung zurückkehren.

Dadurch, dass der Clip von der Halteeinrichtung entnommen wird, bevor die mindestens zwei Halteflächenbereiche in eine vor der Auslenkung bestehende Ausgangstellung zurückkehren, wird eine unerwünschte Reibung zwischen dem Clip und der Halteeinrichtung und somit ein unerwünschter Materialabrieb und eine unerwünschte Verunreinigung des Clips vermieden.

Das erfindungsgemäße Verfahren weist vorzugsweise die vorstehend im Zusammenhang mit der erfindungsgemäßen Clipträgervorrichtung beschriebenen Merkmale und Vorteile auf.

Insbesondere kann das erfindungsgemäße Verfahren den Vorteil aufweisen, dass eine Applikatorzange, mittels welcher der Clip von der Clipträgervorrichtung entnommen wird, nicht über die gesamte Dauer der Entnahme des Clips in Kontakt mit den mindestens zwei Halteflächenbereichen der Halteeinrichtung sein muss, um die mindestens zwei Halteflächenbereiche zum Lösen der kraft- und/oder formschlüssigen Verbindung zwischen dem Clip und der Halteeinrichtung weg zu halten. Dadurch kann eine Beeinträchtigung der Applikatorzange beim Entnehmen des Clips von der Clipträgervorrichtung und insbesondere eine Reibung an der Clipträgervorrichtung vermieden werden.

Die benötigte Zeit für eine Entnahme eines Clips von der Clipträgervorrichtung, insbesondere die Zeit zwischen dem Einführen der Applikatorzange in die Clipträgervorrichtung bis zum vollständigen Verlassen der Applikatorzange mit dem Clip aus der Clipträgervorrichtung, beträgt vorzugsweise zwischen ungefähr 0,5 Sekunden bis ungefähr 2 Sekunden und ist unter anderem abhängig von der Größe des Clips.

Aufgrund der Auslenkung der mindestens zwei Halteflächenbereiche mittels der Applikatorzange zum Entnehmen des Clips von der Clipträgervorrichtung wird vorzugsweise ein Zugriffsbereich vergrößert, sodass ein einfacheres Angreifen der Applikatorzange an dem zu entnehmenden Clip möglich ist.

Bei dem erfindungsgemäßen Verfahren kann vorgesehen sein, dass der Clip zum Lösen der kraft- und/oder formschlüssigen Verbindung zwischen der Halteeinrichtung und dem Clip relativ zu einem Grundkörper der Clipträgervorrichtung entgegen einer Entnahmerichtung verschoben wird. Insbesondere dann, wenn der Clip zumindest näherungsweise hufeisenförmig ausgebildet ist, kann durch die Verschiebung des Clips relativ zu dem Grundkörper der Clipträgervorrichtung entgegen der Entnahmerichtung eine Auslenkung der mindestens zwei Halteflächenbereiche der Halteeinrichtung und somit ein Lösen der kraft- und/oder formschlüssigen Verbindung zwischen dem Clip und der Halteeinrichtung bewirkt werden.

Alternativ oder ergänzend hierzu kann vorgesehen sein, dass der Clip zum Lösen der kraft- und/oder formschlüssigen Verbindung zwischen der Halteeinrichtung und dem Clip relativ zu einem Grundkörper der Clipträgervorrichtung gekippt und/oder gedreht wird.

Ferner kann vorgesehen sein, dass ein Ursprungswinkel, welcher von zwei Schenkeln des Clips eingeschlossen wird, zum Lösen der kraft- und/oder formschlüssigen Verbindung zwischen der Halteeinrichtung und dem Clip verkleinert wird. Eine Öffnungsweite des Clips wird hierbei insbesondere kurzzeitig verringert, sodass die Halteflächenbereiche relativ zueinander ausgelenkt werden.

Besonders günstig ist es, wenn durch die Auslenkung der mindestens zwei Halteflächenbereiche relativ zueinander zum Lösen der kraft- und/oder formschlüssigen Verbindung zwischen der Halteeinrichtung und dem Clip eine Reibwirkung zwischen mindestens einem Halteflächenbereich der Halteeinrichtung und dem Clip bei der Entnahme des Clips um mindestens ungefähr 50 %, beispielsweise um mindestens ungefähr 70 %, insbesondere um mindestens ungefähr 85 %, reduziert, vorzugsweise vollständig vermieden, wird. Auf diese Weise ist ein Materialabrieb von der Halteeinrichtung und somit eine Verunreinigung des Clips bei der Entnahme des Clips von der Halteeinrichtung vorzugsweise ganz vermeidbar.

Die erfindungsgemäße Clipträgervorrichtung eignet sich insbesondere zur Durchführung des erfindungsgemäßen Verfahrens.

Die erfindungsgemäße Clipträgervorrichtung eignet sich insbesondere zur Aufbewahrung eines Vorrats an Ligaturclips, wobei sowohl Standardclips als auch Doppelstegclips, vorzugsweise auf einem gemeinsamen Clipbänkchen, anordenbar sind.

Die erfindungsgemäße Clipträgervorrichtung ist vorzugsweise kostengünstig herstellbar und montierbar. Ferner ist eine Bestückung der erfindungsgemäßen Clipträgervorrichtung mittels mindestens eines Clips besonders einfach durchführbar. Die erfindungsgemäße Clipträgervorrichtung kann beispielsweise als Einwegteil ausgebildet sein.

Insbesondere dann, wenn die Clipträgervorrichtung ein viskoelastisches Material umfasst, welches lediglich eine geringe Haltekraft auf den mindestens einen Clip ausübt, kann vorgesehen sein, dass zur sicheren Lagerung des mindestens einen Clips an der Clipträgervorrichtung während eines Transports der Clipträgervorrichtung eine Verpackung, beispielsweise eine Abdeckung und/oder ein Blister, vorgesehen ist.

Das Freigeben des mindestens einen Clips von der Clipträgervorrichtung als Reaktion auf das Einführen einer Applikatorzange und das Angreifen der Applikatorzange an dem mindestens einen Clip erfolgt vorzugsweise mit einer zeitlichen Abhängigkeit und gerade nicht nur in direktem Zusammenwirken mit der Applikatorzange.

Die zeitliche Abhängigkeit wird insbesondere durch ein träge reagierendes, viskoelastisches Material erreicht, wobei insbesondere das Rückstellen der Halteflächenbereiche, welche beispielsweise an Kontaktelementen angeordnet sind, zum Festlegen des mindestens einen Clips an der Clipträgervorrichtung mit zeitlicher Verzögerung erfolgt.

Zumindest für die Dauer der Entnahme des mindestens einen Clips von der Clipträgervorrichtung wird vorzugsweise ein Formschluss zwischen dem mindestens einen Clip und den Halteflächenbereichen der Clipträgervorrichtung aufgehoben.

Das Maß des Reibschlusses zwischen dem mindestens einen zu haltenden Clip und der Clipträgervorrichtung und/oder die zeitliche Abhängigkeit der Verformung der Clipträgervorrichtung, insbesondere der Auslenkung des mindestens einen Halteflächenbereichs und/oder der Bewegung des mindestens einen Auflageelements der Clipträgervorrichtung, werden vorzugsweise durch eine Abstimmung der Geometrie der Clipträgervorrichtung auf den mindestens einen zuhaltenden Clip, beispielsweise über eine definierte Reibwirkung, welche ein verzögertes Rückstellen bewirken kann, an einem durch die Applikatorzange ausgelenkten Halteelement, realisiert.

Bei einer Ausgestaltung der Erfindung ist vorgesehen, dass die Clipträgervorrichtung einstückig aus einem Kunststoff hergestellt wird und dass das träge Verhalten durch einen zusätzlich angebrachten viskoelastischen Körper realisiert wird. Das träge Verhalten der Halteflächenbereiche wird vorzugsweise über einen zusammenhängenden viskoelastischen Körper, beispielsweise durch eine eingelegte geschäumte Folie, erreicht.

Insbesondere bei der Verwendung von viskoelastischen Materialien kann vorgesehen sein, dass die viskoelastischen Materialien nicht in direktem Oberflächenkontakt mit den Clips stehen, sondern über zwischengeschaltete, weitgehend nicht viskoelastische Elemente wirken. Hierbei ist insbesondere ein Mehrschichtaufbau der Clipträgervorrichtung vorgesehen, wobei sich die einzelnen Schichten in ihrem Materialverhalten, insbesondere hinsichtlich der Viskoelastizität und/oder der Steifigkeit, unterscheiden. Insbesondere kann dabei vorgesehen sein, dass ein geschäumter Werkstoff mit einer zusätzlichen, nicht geschäumten und dennoch verformbaren Deckschicht versehen ist und dass nur diese Deckschicht in direktem Kontakt mit der Oberfläche des Clips steht.

Die erfindungsgemäße Clipträgervorrichtung ist vorzugsweise besonders einfach aufgebaut und umfasst insbesondere keine beweglichen, filigranen Rückhalteglieder.

Insbesondere dann, wenn die Clipträgervorrichtung nachgiebig ausgebaut ist und insbesondere keine steifen Auflageflächen für den mindestens einen Clip umfasst, kann der Clip insbesondere zur Entnahme von der Clipträgervorrichtung in geringem Maße sich an der Applikatorzange ausrichten, sodass eine verbesserte Übergabe des Clips von der Clipträgervorrichtung an die Maulteile der Applikatorzange möglich ist.

Weitere Merkmale und Vorteile der Erfindung sind Gegenstand der nachfolgenden Beschreibung und der zeichnerischen Darstellung von Ausführungsbeispielen.

In den Zeichnungen zeigen:
- Figur 1:: eine schematische Darstellung eines vertikalen Schnitts durch eine Clipträgervorrichtung mit einem daran angeordneten Clip;
- Figur 2:: eine schematische Darstellung des Burgers-Modells zur Veranschaulichung der Viskoelastizität;
- Figur 3:: eine schematische perspektivische Darstellung einer Clipträgervorrichtung mit einem hiervon abgehobenen Ligaturclip;
- Figur 4:: eine schematische perspektivische Darstellung der Clipträgervorrichtung aus Figur 3, wobei eine Applikatorzange an dem Clip zum Abnehmen desselben von der Clipträgervorrichtung angreift;
- Figur 5a:: eine schematische Darstellung eines vertikalen Schnitts durch die Clipträgervorrichtung aus Figur 3 in einem Ursprungszustand;
- Figur 5b:: eine der Figur 5a entsprechende Darstellung der Clipträgervorrichtung aus Figur 3 in einem Lagerzustand, in welchem ein Clip an der Clipträgervorrichtung gehalten ist;
- Figur 5c:: eine der Figur 5a entsprechende Darstellung der Clipträgervorrichtung aus Figur 3, wobei die Clipträgervorrichtung vor einer Entnahme des Clips von der Clipträgervorrichtung zusätzlich deformiert ist;
- Figur 5d:: eine der Figur 5a entsprechende Darstellung der Clipträgervorrichtung aus Figur 3, wobei die gemäß Figur 5c deformierte Clipträgervorrichtung nach dem Entnehmen des Clips von der Clipträgervorrichtung nicht sofort in den Ursprungszustand zurückkehrt;
- Figur 5e:: eine der Figur 5a entsprechende Darstellung der Clipträgervorrichtung aus Figur 3, wobei die Clipträgervorrichtung nach der Entnahme des Clips von der Clipträgervorrichtung teilweise in ihren Ursprungszustand zurückgekehrt ist;
- Figur 5f:: eine der Figur 5a entsprechende Darstellung der Clipträgervorrichtung aus Figur 3, welche nach einer Deformierung durch die Anordnung und Entnahme eines Clips wieder in den Ursprungszustand zurückgekehrt ist;
- Figur 6:: eine der Figur 5b entsprechende Darstellung einer Clipträgervorrichtung mit einem als Doppelsteg ausgebildeten, an der Clipträgervorrichtung angeordneten Clip; und
- Figur 7:: ein Diagramm zur Illustration der Eigenschaften eines viskoelastischen Materials.

Gleiche oder funktional äquivalente Elemente werden in sämtlichen Figuren mit denselben Bezugszeichen bezeichnet.

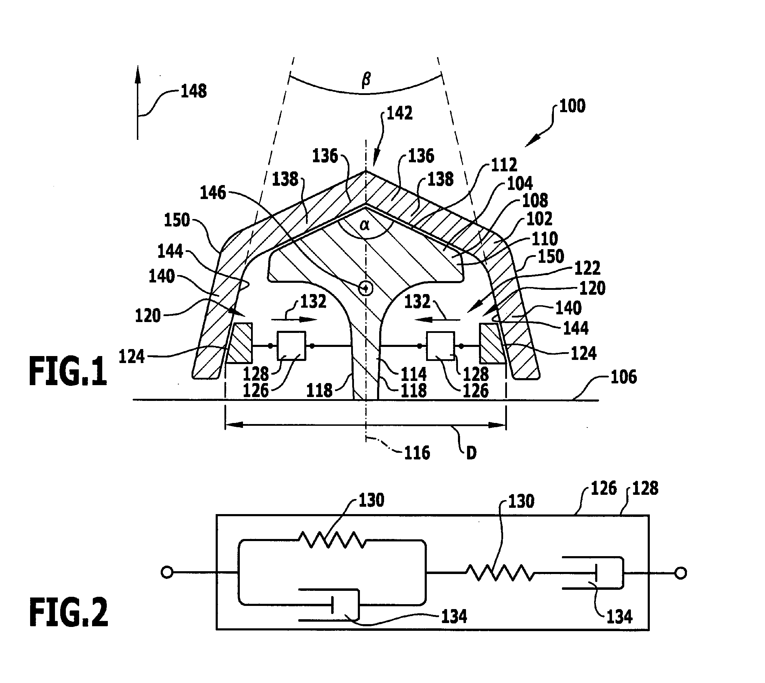

In Figur 1 ist eine medizinische oder chirurgische Clipträgervorrichtung 100 für mindestens einen medizinischen oder chirurgischen Clip 102 dargestellt.

Die Clipträgervorrichtung 100 umfasst einen Grundkörper 104, welcher an einer Grundplatte 106 angeordnet ist.

Der Grundkörper 104 umfasst ein Auflageteil 108, welches ein Auflageelement 110 zum Auflegen mindestens eines Clips 102 auf die Clipträgervorrichtung 100 bildet.

Eine der Grundplatte 106 abgewandte Oberseite des Auflageelements 110 bildet eine Auflagefläche 112 der Clipträgervorrichtung 100 zum Auflegen des Clips 102.

Der Auflageteil 108 des Grundkörpers 104 ist mittels eines stegförmigen Bereichs 114 des Grundkörpers 104 an der Grundplatte 106 der Clipträgervorrichtung 100 angeordnet.

Die Clipträgervorrichtung 100 und damit auch der Grundkörper 104 sind zumindest näherungsweise spiegelsymmetrisch bezüglich einer Längsmittelebene 116 der Clipträgervorrichtung 100 ausgebildet.

Die Längsmittelebene 116 verläuft dabei im Wesentlichen senkrecht zu der Grundplatte 106 der Clipträgervorrichtung 100.

An einander gegenüberliegenden Seitenflächen 118 des stegförmigen Bereichs 114 des Grundkörpers 104 ist jeweils mindestens ein Halteelement 120 zum Halten des Clips 102 angeordnet.

Die Halteelemente 120 bilden zusammen eine Halteeinrichtung 122 der Clipträgervorrichtung 100.

Die dem stegförmigen Bereich 114 des Grundkörpers 104 der Clipträgervorrichtung 100 abgewandten Oberflächen der Halteelemente 120 bilden Halteflächenbereiche 124, mittels welchen die Halteelemente 120 an einem an der Clipträgervorrichtung 100 angeordneten Clip 102 angreifen.

Die Halteelemente 120 umfassen jeweils eine Dämpfungseinrichtung 126 und eine Rückstelleinrichtung 128. Die Halteelemente 120 sind deformierbar ausgebildet, sodass die Halteflächenbereiche 124 aus einer Ausgangsstellung ausgelenkt werden können. Mittels der Rückstelleinrichtungen 128 sind die Halteflächenbereiche 124 in eine vorgegebene Position zurück bringbar.

Die Dämpfungseinrichtungen 126 dienen dabei der Dämpfung der Bewegung, welche die Halteflächenbereiche 124 nach einer Auslenkung aus einer Ausgangsstellung aufgrund der Wirkung der Rückstelleinrichtungen 128 ausführen.

Bei der in Figur 1 dargestellten Clipträgervorrichtung 100 ist insbesondere vorgesehen, dass die Halteflächenbereiche 124 in Richtung des stegförmigen Bereichs 114 des Grundkörpers 104 der Clipträgervorrichtung 100 auslenkbar sind. Mittels der Rückstelleinrichtungen 128 sind die Halteflächenbereiche 124 nach einer solchen Auslenkung von dem stegförmigen Bereich 114 des Grundkörpers 104 der Clipträgervorrichtung 100 weg bewegbar. Die mittels der Rückstelleinrichtungen 128 auf die Halteflächenbereiche 124 zur Bewegung derselben ausgeübte Kraft ist durch die Dämpfungseinrichtungen 126 dämpfbar, sodass die Bewegung der Halteflächenbereiche 124 von der ausgelenkten Stellung in die Ausgangsstellung mit einer langsamen Geschwindigkeit erfolgt.

Die Dämpfungseinrichtung 126 und die Rückstelleinrichtung 128 eines jeden Halteelements 120 sind vorzugsweise einstückig aus demselben Material, insbesondere einem viskoelastischen Material, gebildet.

Die Rückstelleigenschaften und die Dämpfungseigenschaften eines solchen viskoelastischen Materials lassen sich beispielsweise mit dem in Figur 2 dargestellten Burgers-Modell als Kombination des so genannten Kelvin-Voigt-Modells mit dem so genannten Maxwell-Modell beschreiben.

Gemäß dem Kelvin-Voigt-Modell ist eine Feder 130 vorgesehen, welche in einer Belastungsrichtung 132 parallel zu einem Dämpfer 134 angeordnet ist. Dieses System gemäß dem Kelvin-Voigt-Modell ist insgesamt seriell in der Belastungsrichtung 132 an eine weitere Feder 130 und an einen in der Belastungsrichtung seriell geschalteten Dämpfer 134 gemäß dem Maxwell-Modell gekoppelt.

Mittels dieses Systems aus Federn 130 und Dämpfern 134 gemäß dem Burgers-Modell lässt sich das Material, weiches die Halteelemente 120 umfassen oder aus welchem die Halteelemente 120 zumindest teilweise gebildet sind, besonders einfach beschreiben. So führen die Federn 130 nach einer Deformation des viskoelastischen Materials zu der von elastischen Materialien bekannten Rückkehr in den Ausgangszustand. Die Dämpfer 134 dienen hierbei zur Verlangsamung der Rückkehrbewegung, sodass das Material gerade nicht elastisch, sondern viskoelastisch ausgebildet ist.

An der vorstehend beschriebenen Clipträgervorrichtung 100 ist, wie in Figur 1 dargestellt, ein Clip 102 anordenbar.

Der Clip 102 ist vorzugsweise zumindest näherungsweise hufeisenförmig ausgebildet und in einem Lagerzustand an der Clipträgervorrichtung 100 zumindest näherungsweise spiegelsymmetrisch bezüglich der Längsmittelebene 116 der Clipträgervorrichtung 100 ausgebildet.

Der Clip 102 umfasst zwei Schenkel 136, welche jeweils einen Mittelabschnitt 138 und einen Endabschnitt 140 umfassen.

Die Mittelabschnitte 138 der Schenkel 136 des Clips 102 im Lagerzustand des Clips 102 an der Clipträgervorrichtung 100 sind im Bereich der vertikalen Längsmittelebene 116 miteinander verbunden und schließen einen Winkel α von beispielsweise 120° miteinander ein.

Die Endabschnitte 140 der Schenkel 136 des Clips 102 sind an einander abgewandten Enden der Mittelabschnitte 138 der Schenkel 136 angeordnet und schließen einen Winkel β von beispielsweise ungefähr 30° miteinander ein.

Der Clip 102 ist vorzugsweise aus einem metallischen Material gebildet, sodass der Clip 102 eine Rückstelleinrichtung 142 umfasst, welche den Clip 102 nach einer Deformation zumindest näherungsweise in den Ausgangszustand zurückbringt. Insbesondere ist hierbei vorgesehen, dass nach einer Deformation des Clips 102, durch welche der Winkel α, welcher von den Mittelabschnitten 138 der Schenkel 136 des Clips 102 eingeschlossen wird, verkleinert wird, die Rückstelleinrichtung 142 den Clip 102 in die Ausgangsstellung zurückbringt, in welcher der Winkel α den in der Ausgangsstellung vorgegebenen Wert hat.

In dem in Figur 1 dargestellten Lagerzustand des Clips 102 an der Clipträgervorrichtung 100 liegt der Clip 102 mit einer Innenseite 144 im Bereich der Mittelabschnitte 138 der Schenkel 136 des Clips 102 auf der Auflagefläche 112 des Auflageelements 110, das heißt auf der der Grundplatte 106 abgewandten Oberseite des Auflageteils 108 des Grundkörpers 104, auf.

Das Auflageelement 110 ist hierzu vorzugsweise so ausgebildet, dass die Auflagefläche 112 des Auflageelements 110 zumindest näherungsweise eine Form aufweist, welche zumindest näherungsweise komplementär zu einer Form der Innenseite 144 des Clips 102 im Bereich der Mittelabschnitte 138 der Schenkel 136 des Clips 102 ist.

Das Auflageelement 110 ist beispielsweise aus einem elastischen Material gebildet, sodass insbesondere bei der Anordnung des Clips 102 an der Clipträgervorrichtung 100 Handhabungsungenauigkeiten besonders einfach ausgeglichen werden können.

In dem in Figur 1 dargestellten Zustand des Clips 102 an der Clipträgervorrichtung 100 liegen die Halteelemente 120 mit den Halteflächenbereichen 124 an den Innenseiten 144 des Clips 102 im Bereich der Endabschnitte 140 der Schenkel 136 des Clips 102 an.

Da die Halteflächenbereiche 124 in dem in Figur 1 dargestellten Lagerzustand des Clips 102 an der Clipträgervorrichtung 100 nicht in ihrer Ursprungsstellung, in welcher sie ihren maximalen Abstand von der Längsmittelebene 116 haben, angeordnet sind, sondern vielmehr durch die Endabschnitte 140 der Schenkel 136 des Clips 102 in Richtung der Längsmittelebene 116 ausgelenkt sind, bewirkt die Rückstelleinrichtung 128 eine von der Längsmittelebene 116 weg gerichtete Kraft, welche die Halteflächenbereiche 124 an die Innenseite 144 des Clips 102 im Bereich der Endabschnitte 140 der Schenkel 136 des Clips 102 drückt. Auf diese Weise wird eine Reibwirkung erzeugt, welche den Clip 102 mittels einer zumindest reibschlüssigen, vorzugsweise insbesondere mittels einer formschlüssigen, Verbindung an der Clipträgervorrichtung 100 hält.

Die Clipträgervorrichtung 100 erstreckt sich entlang einer in der Längsmittelebene 116 und parallel zu der Grundplatte 106 verlaufenden Längsachse 146 über eine Länge von beispielsweise 2 bis 5 cm, sodass mehrere, beispielsweise 8 oder 10 Clips 102 nebeneinander gereiht an der Clipträgervorrichtung 100 angeordnet werden können.

Die in Figur 1 dargestellte Clipträgervorrichtung 100 wird wie folgt benutzt:

Nach der Herstellung der Clipträgervorrichtung 100 und vor der Anordnung von Clips 102 an der Clipträgervorrichtung 100 sind die Halteflächenbereiche 124 der Halteelemente 102 der Clipträgervorrichtung 100 in einer Ursprungsstellung angeordnet, in welcher ein Abstand D der Halteflächenbereiche 124 voneinander maximal ist.

Insbesondere ist der Abstand D der Halteflächenbereiche 124 voneinander in der Ursprungsstellung größer als der Abstand der Endabschnitte 140 der Schenkel 136 des Clips 102, an welchen die Halteflächenbereiche 124 im Lagerzustand des Clips 102 an der Clipträgervorrichtung 100 angreifen.

Zur Bestückung der Clipträgervorrichtung 100 mit einem Clip 102 wird der Clip 102 entgegen einer senkrecht zu der Grundplatte 106 verlaufenden Entnahmerichtung 148 mit seiner Innenseite 144 auf die Clipträgervorrichtung 100 aufgesetzt.

Hierbei kommt der Clip 102 mit der Innenseite 144 im Bereich der Mittelabschnitte 138 der Schenkel 136 des Clips 102 mit der Auflagefläche 112 des Auflageelements 110 der Clipträgervorrichtung 100 zur Anlage.

Aufgrund des vergleichsweise geringen Abstands der Endabschnitte 140 der Schenkel 136 des Clips 102 in den Bereichen, in welchen die Halteflächenbereiche 124 an der Innenseite 144 des Clips 102 angreifen, werden die Halteelemente 120 beim Aufsetzen des Clips 102 auf die Clipträgervorrichtung 100 deformiert.

Die Halteflächenbereiche 124 werden dabei in Richtung der Längsmittelebene 116 ausgelenkt, sodass der Abstand D der Halteflächenbereiche 124 voneinander verkleinert wird.

Die Rückstelleinrichtungen 128 der Halteelemente 120 bewirken anschließend eine Kraft, welche die Halteflächenbereiche 124 von der Längsmittelebene 116 weg bewegt und im Bereich der Endabschnitte 140 der Schenkel 136 des Clips 102 an die Innenseite 144 des Clips 102 anpresst.

Die mittels der Rückstelleinrichtung 128 auf den Clip 102 übertragene Kraft bewirkt eine reibschlüssige Verbindung des Clips 102 mit der Clipträgervorrichtung 100.

Die Dämpfungseinrichtung 126 bewirkt eine Verlangsamung der Rückstellung der Halteflächenbereiche 124 mittels der Rückstelleinrichtungen 128, sodass beim Aufsetzen des Clips 102 auf die Clipträgervorrichtung 100 eine Reibwirkung zwischen den Halteflächenbereichen 124 und der Innenseite 144 des Clips 102 und somit auch ein Materialabrieb von den Halteflächenbereichen 124 mittels des Clips 102 durch eine zusätzliche Auslenkung der Halteflächenbereiche 124 minimiert werden kann.

Die Entnahme des Clips 102 von der Clipträgervorrichtung 100 funktioniert wie folgt:
Mittels einer (in Figur 1 nicht dargestellten) Applikatorzange greift ein Arzt, welcher den Clip 102 von der Clipträgervorrichtung 100 entnehmen möchte, an einer der Innenseite 144 des Clips 102 abgewandten Außenseite 150 des Clips 102 an.

Hierbei deformiert er den Clip 102 vorzugsweise so, dass der Winkel α, welcher von den Mittelabschnitten 138 des Clips 102 eingeschlossen wird, verkleinert wird und sich so die Endabschnitte 140 der Schenkel 136 des Clips 102 aufeinander zu bewegen.

Hierdurch werden die Halteflächenbereiche 124 aufeinander zu bewegt, das heißt der Abstand D zwischen den Halteflächenbereichen 124 wird verringert.

Sobald die die Deformation des Clips 102 bewirkende Kraft von dem Arzt nicht mehr aufgebracht wird, bewirkt die Rückstelleinrichtung 142 des Clips 102, dass die Mittelabschnitte 138 der Schenkel 136 des Clips 102 den in der Ausgangsstellung eingeschlossenen, vorgegebenen Winkel α einschließen und die Endabschnitte 140 der Schenkel 136 des Clips 102 sich wieder voneinander entfernen.

Die Rückstelleinrichtung 142 des Clips 102 wirkt nicht mit einer Dämpfungseinrichtung zusammen, sodass die Rückstellung des Clips 102 nach dem Lösen der die Deformation bewirkenden Kraft im Wesentlichen instantan erfolgt.

Da die Halteelemente 120 jedoch eine Dämpfungseinrichtung 126 aufweisen, welche der Wirkung der Rückstelleinrichtung 128 der Halteelemente 120 entgegenwirkt, erfolgt die Rückstellung der Halteflächenbereiche 124 nicht instantan, sondern zeitverzögert.

Das unterschiedliche Rückstellen des Clips 102 und der Halteelemente 120 in die vor der Auslenkung bestehende Stellung bewirkt ein Lösen der Halteflächenbereiche 124 von der Innenseite 144 des Clips 102.

Die reibschlüssige Verbindung zwischen dem Clip 102 und der Clipträgervorrichtung 100 ist damit aufgehoben.

Der Clip 102 ist dadurch zumindest solange besonders einfach von der Clipträgervorrichtung 100 entnehmbar, bis die Rückstelleinrichtung 128 die Halteflächenbereiche 124 langsam in die Ausgangsstellung zurückgebracht hat.

Bei einer (in Figur 1 nicht dargestellten) entsprechenden geometrischen Ausgestaltung des Clips 102 und/oder der Halteelemente 120 ist auch denkbar, dass die Halteelemente 120 den Clip 102 mittels einer formschlüssigen Verbindung an der Clipträgervorrichtung 100 halten.

Insbesondere dann, wenn der Clip 102 lediglich mittels einer reibschlüssigen Verbindung an der Clipträgervorrichtung 100 gehalten ist, kann eine (nicht dargestellte) Verpackung, beispielsweise ein Blister vorgesehen sein, welche den Clip 102 zum Transport der Clipträgervorrichtung 100 mit dem Clip 102 zusätzlich festlegt.

Vor der Verwendung der Clipträgervorrichtung 100 und der Entnahme des Clips 102 von der Clipträgervorrichtung 100 wird eine solche Verpackung dann entfernt.

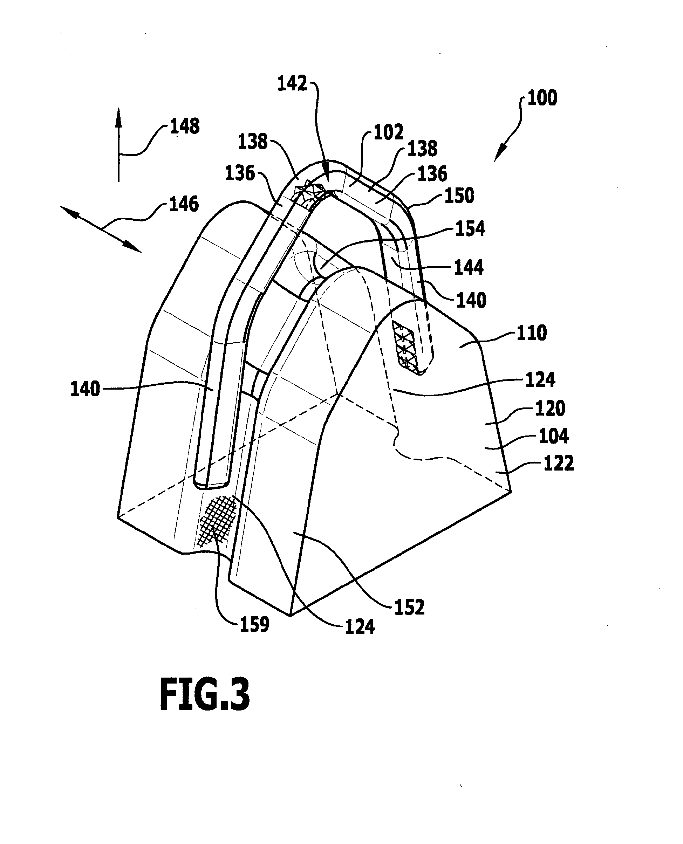

Eine in Figur 3 dargestellte Clipträgervorrichtung 100 unterscheidet sich von der in Figur 1 dargestellten Clipträgervorrichtung 100 dadurch, dass das Auflageelement 110 und die Halteelemente 120 einstückig ausgebildet sind.

Der Grundkörper 104, welcher folglich die Halteelemente 120 und das Auflageelement 110 umfasst, ist vorzugsweise vollständig aus einem viskoelastischen Material gebildet.

Eine Oberfläche 152 des Grundkörpers 104 weist vorzugsweise einen in einer Ebene senkrecht zu der Längsachse 146 genommenen zumindest näherungsweise hufeisenförmigen Querschnitt auf, sodass die Oberfläche 152 des Grundkörpers 104 zumindest näherungsweise eine Form aufweist, welche zumindest näherungsweise einer Form der Innenseite 144 des Clips 102 entspricht.

Bestückt man die in Figur 3 dargestellte Clipträgervorrichtung 100 mit dem Clip 102, das heißt setzt man den Clip 102 entgegen der Entnahmerichtung 148 auf die Clipträgervorrichtung 100 auf, so wird der Grundkörper 104 der Clipträgervorrichtung 100 so deformiert, dass sich eine Vertiefung 154 in der Oberfläche 152 des Grundkörpers 104 bildet, deren Form zumindest näherungsweise komplementär zu der Innenseite 144 des Clips 102 ausgebildet ist.

Die Oberfläche 152 des Grundkörpers 104 bildet dann im Bereich der Vertiefung 154 einerseits die Halteflächenbereiche 124 der Halteelemente 120 der Clipträgervorrichtung 100 und andererseits auch die Auflagefläche 112 des Auflageelements 110 der Clipträgervorrichtung 100.

Aufgrund der viskoelastischen Eigenschaften des Materials des Grundkörpers 104 dehnt sich der Grundkörper 104 nach der Deformation durch das Aufsetzten des Clips 102 soweit aus, bis sich die Oberfläche 152 im Bereich der Vertiefung 154 an die Innenseite 144 des Clips 102 anschmiegt.

Aufgrund der Rückstelleinrichtungen 128 der Halteelemente 120, welche bei der in Figur 3 dargestellten Clipträgervorrichtung 100 durch das viskoelastische Material des Grundkörpers 104 realisiert sind, wird die Oberfläche 152 im Bereich der Vertiefung 154 an die Innenseite 144 des Clips 102 gepresst, sodass der Clip 102 mittels einer reibschlüssigen Verbindung an der Clipträgervorrichtung 100 gehalten wird.

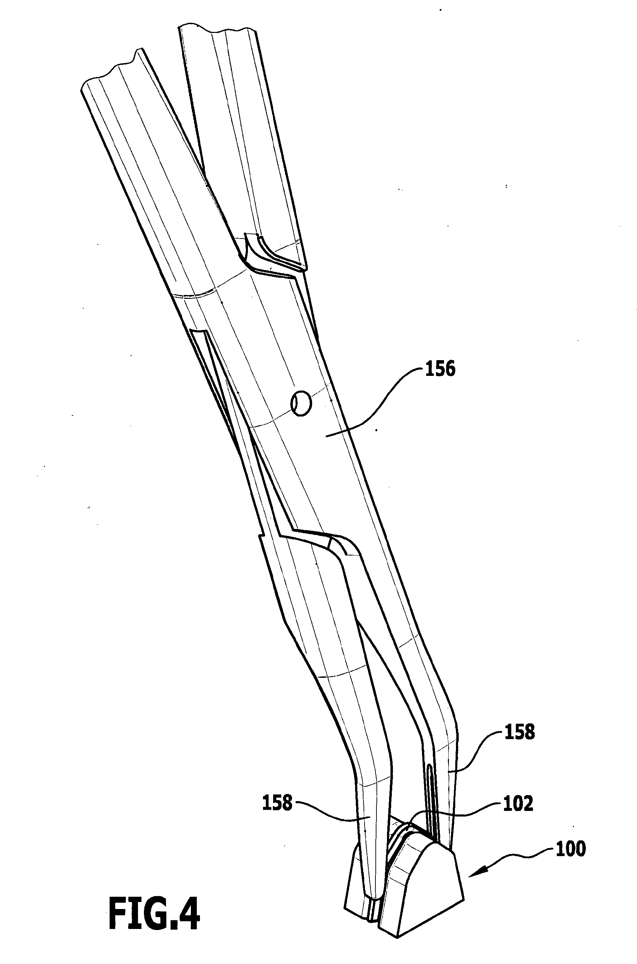

Mittels einer (in Figur 4 dargestellten) Applikatorzange 156 ist der Clip 102 zur Verwendung durch einen Arzt während einer Operation von der Clipträgervorrichtung 100 abnehmbar.

Hierzu greift der Arzt mit Maulteilen 158 der Applikatorzange 156 an den Außenseiten 150 des Clips 102 an und deformiert so den Clip in der Art, dass der von den Mittelabschnitten 138 der Schenkel 136 des Clips 102 eingeschlossene Winkel α verkleinert wird und sich die Endabschnitte 140 der Schenkel 136 des Clips 102 aneinander annähern.

Insbesondere dann, wenn der Clip 102, wie in Figur 3 dargestellt, eine im Lagerzustand des Clips 102 an der Clipträgervorrichtung 100 entgegen der Entnahmerichtung 148 offene V-Form aufweist, kann der Clip zur Entnahme von der Clipträgervorrichtung 100 auch entgegen der Entnahmerichtung 148 auf den Grundkörper 104 gedrückt werden.

In beiden Fällen, das heißt entweder durch die Deformation des Clips 102 oder durch das Verschieben des Clips 102 entgegen der Entnahmerichtung 148 auf den Grundkörper 104 der Clipträgervorrichtung 100 wird eine Auslenkung der Halteflächenbereiche 124 der Halteelemente 120 der Clipträgervorrichtung 100 aufeinander zu bewirkt.

Da sowohl das Zurückstellen des Winkels α in die Ausgangsstellung mittels der Rückstelleinrichtung 142 als auch ein Abheben des Clips 102 in der Entnahmerichtung 148 von der Clipträgervorrichtung 100 im Wesentlichen instantan erfolgen und sich die Halteflächenbereiche 124 nach der durch die Deformation des Clips 102 bewirkten Auslenkung aufgrund der Wirkung der Dämpfungseinrichtungen 126 der Halteelemente 120 zeitverzögert in die Ausgangsstellung zurück bewegen, bewirkt die Deformation des Clips beziehungsweise die Bewegung des Clips 102 entgegen der Entnahmerichtung 148 ein Lösen der reibschlüssigen Verbindung des Clips 102 mit der Clipträgervorrichtung 100, sodass der Clip 102 besonders einfach von der Clipträgervorrichtung 100 entnommen werden kann und insbesondere keinerlei Abrieb von den Halteflächenbereichen 124 der Clipträgervorrichtung 100 und damit keine Verunreinigung des Clips 102 erfolgen.

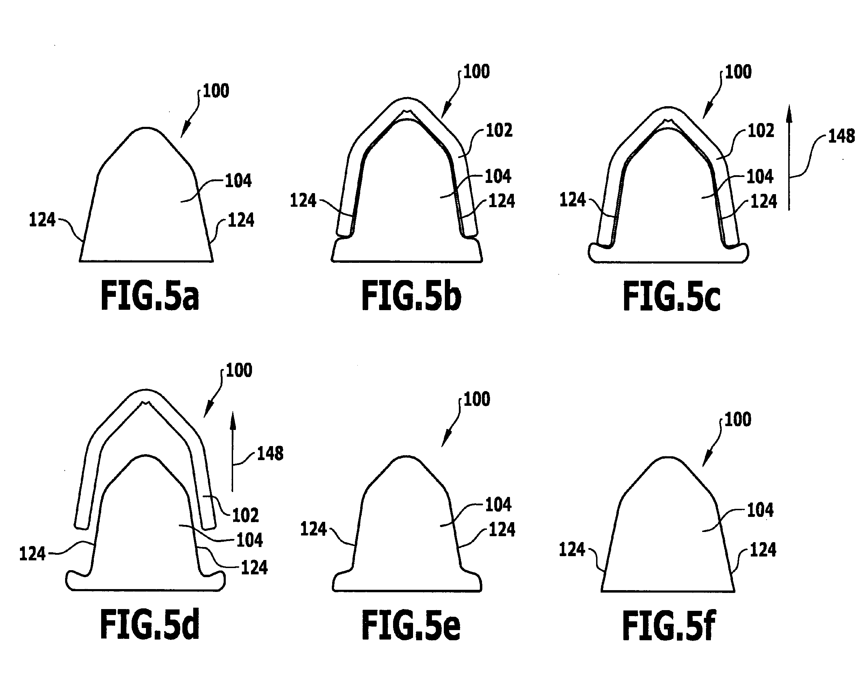

Die unterschiedlichen Stadien der Benutzung der Clipträgervorrichtung 100 gemäß der in den Figuren 3 und 4 dargestellten Ausführungsform sind in den Figuren 5a bis 5f dargestellt.

So zeigt Figur 5a eine Ursprungsstellung des Grundkörpers 104 der Clipträgervorrichtung 100, in welcher der Grundkörper 104 keinerlei Deformationen aufweist. Die Halteflächenbereiche 124 weisen in dieser Ursprungsstellung einen maximalen Abstand D voneinander auf.

In Figur 5b ist eine Lagerstellung der Clipträgervorrichtung 100 dargestellt, in welcher der Grundkörper 104 der Clipträgervorrichtung 100 durch die Bestückung der Clipträgervorrichtung 100 mit einem Clip 102 so deformiert ist, dass der Clip 102 mittels einer reibschlüssigen Verbindung an dem Grundkörper 104 der Clipträgervorrichtung 100 gehalten ist. Die Oberfläche 152 des Grundkörpers 104 liegt dabei im Bereich der Vertiefung 154 direkt an der Innenseite 144 des Clips 102 an und bewirkt so eine reibschlüssige Verbindung zwischen dem Clip 102 und der Clipträgervorrichtung 100.

In Figur 5c ist eine zusätzliche Deformation des Grundkörpers 104 der Clipträgervorrichtung 100 dargestellt, welche sich ergibt, wenn der Clip 102 vor einer Entnahme entgegen der Entnahmerichtung 148 bewegt wird. Der Grundkörper 104 der Clipträgervorrichtung 100 wird dabei entgegen der Entnahmerichtung 148 gestaucht. Hierdurch werden die Halteflächenbereiche 124 der Halteelemente 120 der Clipträgervorrichtung 100 aufeinander zu ausgelenkt, sodass die reibschlüssige Verbindung zwischen dem Clip 102 und der Clipträgervorrichtung 100 aufgehoben wird und der Clip 102 besonders einfach und ohne Materialabrieb von dem Grundkörper 104 der Clipträgervorrichtung 100 entnommen werden kann.

In Figur 5d ist die Clipträgervorrichtung 100 mit dem Clip 102 nach dem Entnehmen des Clips 102 von der Clipträgervorrichtung 100 dargestellt, wobei deutlich wird, dass der Grundkörper 104 nach dem Entnehmen des Clips 102 von der Clipträgervorrichtung 100 nicht sofort in den Ursprungszustand zurückkehrt und somit ein einfaches Abheben des Clips 102 von dem Grundkörper 104 der Clipträgervorrichtung 100 erschwert. Vielmehr verbleibt der Grundkörper 104 der Clipträgervorrichtung 100 aufgrund der viskoelastischen Eigenschaften des Grundkörpers 104 zunächst kurz in der deformierten Stellung und kehrt aufgrund der Wirkung der Dämpfungseinrichtungen 126, welche durch das viskoelastische Material des Grundkörpers 104 realisiert sind, sehr langsam nach Einnehmen eines Übergangszustands (in Figur 5e dargestellt) in die Ursprungsstellung (in Figur 5f dargestellt) zurück.

Insbesondere dann, wenn der Grundkörper 104 der Clipträgervorrichtung 100 zumindest näherungsweise vollständig aus einem viskoelastischen Material gebildet ist, kann vorgesehen sein, dass der Grundkörper 104 mit einer (in Figur 3 angedeuteten) Deckschicht 159 versehen ist, um einen direkten Kontakt zwischen dem viskoelastischen Material des Grundkörpers 104 der Clipträgervorrichtung 100 und dem Clip 102 zu vermeiden. Auf diese Weise kann ein besonders vorteilhaftes viskoelastisches Material gewählt werden, ohne auf die besondere Reinheit, welche für die spätere Verwendung des Clips 102 notwendig ist, Rücksicht nehmen zu müssen.

Im Übrigen stimmt die in den Figuren 3, 4, 5a bis 5f dargestellte Clipträgervorrichtung 100 hinsichtlich Aufbau und Funktion mit der in Figur 1 dargestellten Clipträgervorrichtung 100 überein, auf deren vorstehende Beschreibung insoweit Bezug genommen wird.

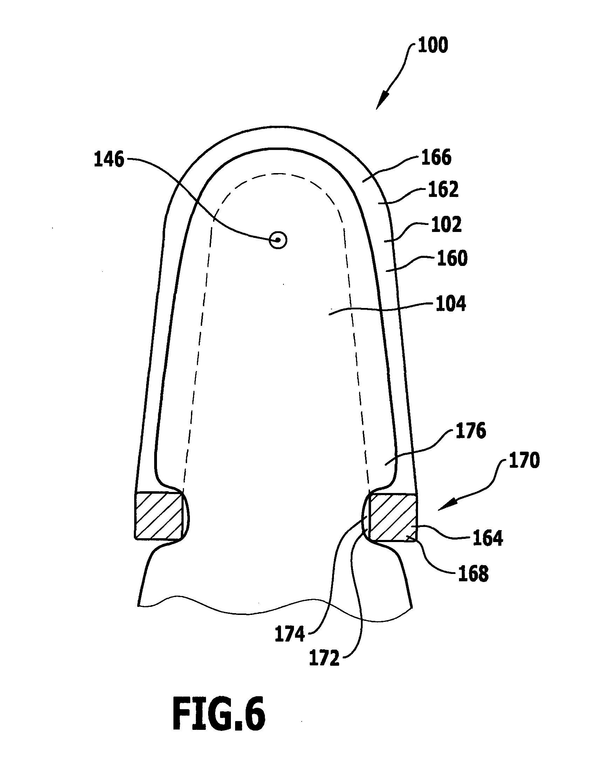

Eine in Figur 6 dargestellte Clipträgervorrichtung 100 unterscheidet sich von der in den Figuren 3, 4, 5a bis 5f dargestellten Clipträgervorrichtung 100 dadurch, dass der Grundkörper 104 der Clipträgervorrichtung 100 einen in einer senkrecht zu der Längsachse 146 ausgerichteten Ebene genommenen im Wesentlichen parabelförmigen Querschnitt aufweist. Die in Figur 6 dargestellte Clipträgervorrichtung 100 eignet sich insbesondere zur Anordnung von als Doppelstegclips 160 ausgebildeten Clips 102.

Derartige, beispielsweise aus der DE 10 2006 001 344 A1 bekannte, Doppelstegclips 160 sind im Wesentlichen aus zwei wie vorstehend beschriebenen hufeisenförmigen Clips 102, welche jeweils einen Steg 162 des Doppelstegclips 160 bilden, gebildet. Die Stege 162 sind dabei an ihren den Mittelabschnitten 138 abgewandten Enden der Endabschnitte 140 der Schenkel 136 in Verbindungsbereichen 164 miteinander verbunden.

Zwischen den parallel zueinander verlaufenden, hufeisenförmigen Stegen 162 des Doppelstegclips 160 ist ein Spalt 166 gebildet, in welchen sich der Grundkörper 104 der Clipträgervorrichtung 100 hinein ausdehnen kann, wenn der Doppelstegclip 160 auf den Grundkörper 104 der Clipträgervorrichtung 100 aufgesetzt wird. Die Darstellung in Figur 6 zeigt einen schematischen vertikalen Schnitt senkrecht zu der Längsachse 146 durch die Clipträgervorrichtung 100 mit dem Doppelstegclip 160 im Bereich des Spalts 166 zwischen den Stegen 162.

Aufgrund der Ausdehnung des Grundkörpers 104 der Clipträgervorrichtung 100 in den Spalt 166 des Doppelstegclips 160 ist, wie in Figur 6 dargestellt, eine formschlüssige Verbindung zwischen dem Doppelstegclip 160 und dem Grundkörper 104 der Clipträgervorrichtung 100 möglich.

Der Verbindungsbereich 164 des Doppelstegclips 160 bildet dabei ein erstes Kopplungselement 168 einer Kopplungseinrichtung 170, welches in einer ein zweites Kopplungselement 172 bildenden, als Ausnehmung ausgebildeten Aufnahme 174 des Grundkörpers 104 angeordnet ist.

Bei der in Figur 6 dargestellten Kopplungseinrichtung 170 wird die formschlüssige Verbindung des Clips 102 mit der Clipträgervorrichtung 100 durch einen in der Entnahmerichtung 148 vor der Aufnahme 174 angeordneten, sich in den Spalt 166 des Doppelstegclips 160 hinein erstreckenden Vorsprung 176 bewirkt.

Insbesondere dann, wenn die geometrische Form des Clips 102 und/oder die geometrische Form der Halteelemente 120 eine formschlüssige Verbindung des Clips 102 mit der Clipträgervorrichtung 100 ermöglichen, kann eine im Lagerzustand des Clips 102 an der Clipträgervorrichtung 100 auf den Clip 102 wirkende Kraft, welche eine reibschlüssige Verbindung zwischen dem Clip 102 und der Clipträgervorrichtung 100 bewirkt, entbehrlich sein.

Insbesondere kann so bei der in Figur 6 dargestellten Clipträgervorrichtung 100 vorgesehen sein, dass der Grundkörper 104 lediglich im Bereich des Vorsprungs 176 aus einem viskoelastischen Material gebildet ist, weil bereits durch das Ausdehnen des Grundkörpers 104 im Bereich des Vorsprungs 176 ein fester Halt des Clips 102 an der Clipträgervorrichtung 100 gewährleistet ist.

Im Übrigen stimmt die in Figur 6 dargestellte Clipträgervorrichtung 100 hinsichtlich Aufbau und Funktion mit der in den Figuren 3, 4, 5a bis 5f dargestellten Clipträgervorrichtung 100 überein, auf deren vorstehende Beschreibung insoweit Bezug genommen wird.

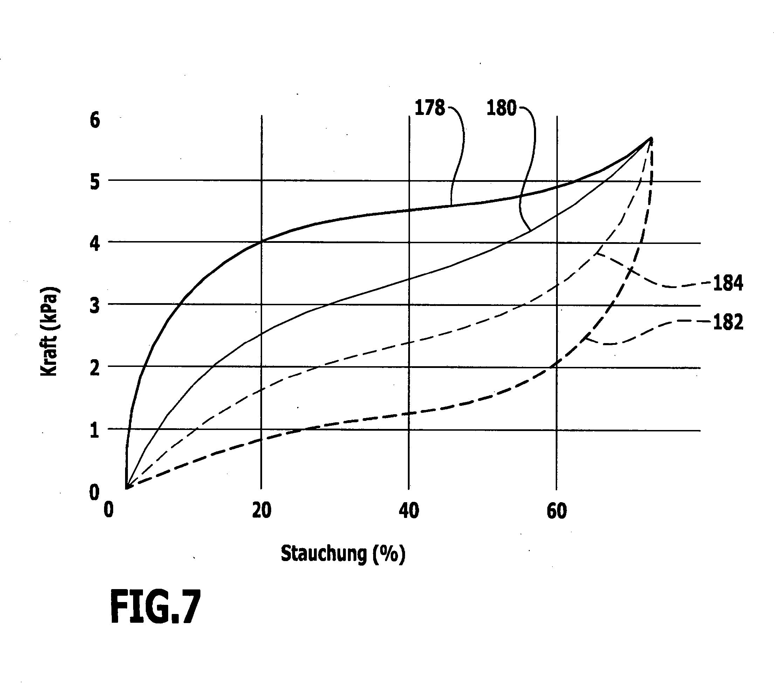

Zur Verdeutlichung der mechanischen Eigenschaften des viskoelastischen Materials zur Verwendung als Material für die Clipträgervorrichtung 100 ist in dem in Figur 7 dargestellten Diagramm die Kraft über der Stauchung des Materials aufgetragen.

Als Vergleichsmaterial zum Vergleich der Eigenschaften wurde ein Standard-Schaumstoff verwendet.

Wie Figur 7 zu entnehmen ist, ist eine Belastungskurve 178 des viskoelastischen Materials deutlich oberhalb einer Belastungskurve 180 des Standard-Schaumstoffs angeordnet. Dies bedeutet, dass im Vergleich zur Stauchung des Standard-Schaumstoffs zur Stauchung des viskoelastischen Materials eine wesentlich höhere Kraft notwendig ist.

Die unterschiedliche Rückverformung in den Ursprungszustand ergibt sich aus einer Entlastungskurve 182 des viskoelastischen Materials, welche deutlich unterhalb einer Entlastungskurve 184 des Standard-Schaumstoffs angeordnet ist.

Die bei der Belastung und bei der Entlastung auftretende große Hysterese der Belastungskurve 178 und der Entlastungskurve 182 des viskoelastischen Materials gibt dabei das durch die mindestens eine Dämpfungseinrichtung 126 bewirkte langsame Rückstellen in die ursprüngliche Form wieder.

Dadurch, dass die mindestens eine Halteeinrichtung 122 mindestens eine der mindestens einen Rückstelleinrichtung 128 entgegenwirkende Dämpfungseinrichtung 126 zur Dämpfung einer Bewegung der mindestens zwei Halteflächenbereiche 124 relativ zueinander von der ausgelenkten Stellung in Richtung der Ausgangsstellung zurück umfasst, ist eine besonders einfache Entnahme des Clips 102 von der Clipträgervorrichtung 100 möglich.

## Patentansprüche

1. Medizinische oder chirurgische Clipträgervorrichtung (100), umfassend mindestens eine Halteeinrichtung (122) zum Halten mindestens eines medizinischen oder chirurgischen Clips (102), welche Halteeinrichtung (122) mindestens zwei relativ zueinander bewegliche Halteflächenbereiche (124) zum kraft- und/oder formschlüssigen Halten des mindestens einen Clips (102) umfasst, wobei die mindestens eine Halteeinrichtung (122) mindestens eine Rückstelleinrichtung (128) zum Bewegen der mindestens zwei Halteflächenbereiche (124) relativ zueinander in eine kraftneutrale Stellung, in welcher die mindestens eine Rückstelleinrichtung (128) die mindestens zwei Halteflächenbereiche (124) in einer vorgegebenen Stellung relativ zueinander hält, nach einer Auslenkung der mindestens zwei Halteflächenbereiche (124) relativ zueinander aus einer Ausgangsstellung in eine ausgelenkte Stellung umfasst, **dadurch gekennzeichnet, dass** die mindestens eine Halteeinrichtung (122) mindestens eine der mindestens einen Rückstelleinrichtung (128) entgegenwirkende Dämpfungseinrichtung (126) zur Dämpfung einer Bewegung der mindestens zwei Halteflächenbereiche (124) relativ zueinander von der ausgelenkten Stellung in Richtung der Ausgangsstellung zurück umfasst.

2. Clipträgervorrichtung nach Anspruch 1, **dadurch gekennzeichnet, dass** die Clipträgervorrichtung (100) einen Grundkörper (104) umfasst und dass mindestens ein Halteflächenbereich (124) relativ zu dem Grundkörper (104) beweglich ausgebildet ist.

3. Clipträgervorrichtung nach einem der Ansprüche 1 oder 2, **dadurch gekennzeichnet, dass** die mindestens eine Halteeinrichtung (122) eine Auflagefläche (112) für den mindestens einen Clip (102) umfasst.

4. Clipträgervorrichtung nach Anspruch 3, **dadurch gekennzeichnet, dass** die Clipträgervorrichtung (100) mindestens ein Auflageelement (110) umfasst, dessen Oberfläche zumindest einen Teil der Auflagefläche (112) für den mindestens einen Clip (102) bildet.

5. Clipträgervorrichtung nach einem der Ansprüche 1 bis 4, **dadurch gekennzeichnet, dass** die mindestens eine Rückstelleinrichtung (128) und die mindestens eine Dämpfungseinrichtung (126) separate Bauteile der mindestens einen Halteeinrichtung (122) sind oder dass die mindestens eine Rückstelleinrichtung (128) einstückig mit der mindestens einen Dämpfungseinrichtung (126) ausgebildet ist.

6. Clipträgervorrichtung nach einem der Ansprüche 1 bis 5, **dadurch gekennzeichnet, dass** die mindestens eine Rückstelleinrichtung (128) und die mindestens eine Dämpfungseinrichtung (126) aus demselben Material gebildet sind.

7. Clipträgervorrichtung nach Anspruch 6, **dadurch gekennzeichnet, dass** das Material der mindestens einen Rückstelleinrichtung (128) und der mindestens einen Dämpfungseinrichtung (126) ein viskoelastisches Material ist.

8. Clipträgervorrichtung nach Anspruch 7, **dadurch gekennzeichnet, dass** das viskoelastische Material ein Material ist, welches nach einer Verformung im Wesentlichen vollständig in einen Ursprungszustand zurückkehrt.

9. Clipträgervorrichtung nach Anspruch 7 oder 8, **dadurch gekennzeichnet, dass** das viskoelastische Material eine Relaxationszeit, welche nach einer Deformation des viskoelastischen Materials bis zur zumindest teilweisen Wiederherstellung eines Ursprungszustands vergeht, von mindestens ungefähr 0,5 Sekunden aufweist und/oder von höchstens ungefähr 2 Sekunden aufweist.

10. Clipträgervorrichtung nach einem der Ansprüche 7 bis 9, **dadurch gekennzeichnet, dass** die mindestens eine Halteeinrichtung (122) ein viskoelastisches Material umfasst oder aus einem viskoelastischen Material gebildet ist.

11. Clipträgervorrichtung nach einem der Ansprüche 1 bis 10, **dadurch gekennzeichnet, dass** die Clipträgervorrichtung (100) mindestens einen Clip (102) umfasst.

12. Clipträgervorrichtung nach Anspruch 11, **dadurch gekennzeichnet, dass** die Clipträgervorrichtung (100) mindestens eine Kopplungseinrichtung (170) zum Koppeln des mindestens einen Clips (102) mit der mindestens einen Halteeinrichtung (122) umfasst.

13. Verfahren zum Entnehmen eines medizinischen oder chirurgischen Clips (102) von einer Halteeinrichtung (122) einer medizinischen oder chirurgischen Clipträgervorrichtung (100) für mindestens einen medizinischen oder chirurgischen Clip (102), bei welchem mindestens zwei Halteflächenbereiche (124) der Halteeinrichtung (122) zum Lösen einer kraft- und/oder formschlüssigen Verbindung zwischen dem Clip (102) und der Halteeinrichtung (122) relativ zueinander ausgelenkt werden, wobei die mindestens eine Halteeinrichtung (122) mindestens eine Rückstelleinrichtung (128) zum Bewegen der mindestens zwei Halteflächenbereiche (124) relativ zueinander in eine kraftneutrale Stellung, in welcher die mindestens eine Rückstelleinrichtung (128) die mindestens zwei Halteflächenbereiche (124) in einer vorgegebenen Stellung relativ zueinander hält, nach einer Auslenkung der mindestens zwei Halteflächenbereiche (124) relativ zueinander aus einer Ausgangsstellung in eine ausgelenkte Stellung umfasst, **dadurch gekennzeichnet, dass** die mindestens eine Halteeinrichtung (122) mindestens eine der mindestens einen Rückstelleinrichtung (128) entgegenwirkende Dämpfungseinrichtung (126) zur Dämpfung einer Bewegung der mindestens zwei Halteflächenbereiche (124) relativ zueinander von der ausgelenkten Stellung in Richtung der Ausgangsstellung zurück umfasst und dass der Clip (102) von der Halteeinrichtung (122) entnommen wird, bevor die mindestens zwei Halteflächenbereiche (124) in eine vor der Auslenkung bestehende Ausgangsstellung zurückkehren.

14. Verfahren nach Anspruch 13, **dadurch gekennzeichnet, dass** durch die Auslenkung der mindestens zwei Halteflächenbereiche (124) relativ zueinander zum Lösen der kraft- und/oder reibschlüssigen Verbindung zwischen der Halteeinrichtung (122) und dem Clip (102) eine Reibwirkung zwischen mindestens einem Halteflächenbereich (124) der Halteeinrichtung (122) und dem Clip (102) bei der Entnahme des Clips (102) um mindestens ungefähr 50 % reduziert wird.

15. Verwendung einer Clipträgervorrichtung (100) nach einem der Ansprüche 1 bis 12 zur Durchführung eines Verfahrens nach einem der Ansprüche 13 oder 14.

## Claims

1. Medical or surgical clip holder (100), comprising at least one holding device (122) for holding at least one medical or surgical clip (102), the at least one holding device (122) comprising at least two holding surface areas (124) movable relative to each other for holding the at least one clip (102) with force locking and/or positive locking, wherein the at least one holding device (122) comprises at least one restoring device (128) for moving the at least two holding surface areas (124) relative to each other into a force-neutral position in which the at least one restoring device (128) holds the at least two holding surface areas (124) in a predefined position relative to each other following deflection of the at least two holding surface areas (124) relative to each other from an initial position into a deflected position, **characterized in that** the at least one holding device (122) comprises at least one damping device (126) counteracting the at least one restoring device (128) for damping a movement of the at least two holding surface areas (124) relative to each other from the deflected position back in the direction of the initial position.

2. Clip holder in accordance with claim 1, **characterized in that** the clip holder (100) comprises a main body (104) and **in that** at least one holding surface area (124) is configured so as to be movable relative to the main body (104).

3. Clip holder in accordance with claim 1 or 2, **characterized in that** the at least one holding device (122) comprises a supporting surface (112) for the at least one clip (102).

4. Clip holder in accordance with claim 3, **characterized in that** the clip holder (100) comprises at least one supporting element (110), the surface of which forms at least part of the supporting surface (112) for the at least one clip (102).

5. Clip holder in accordance with any one of claims 1 to 4, **characterized in that** the at least one restoring device (128) and the at least one damping device (126) are separate components of the at least one holding device (122) or **in that** the at least one restoring device (128) is formed in one piece with the at least one damping device (126).

6. Clip holder in accordance with any one of claims 1 to 5, **characterized in that** the at least one restoring device (128) and the at least one damping device (126) are made of the same material.

7. Clip holder in accordance with claim 6, **characterized in that** the material of the at least one restoring device (128) and of the at least one damping device (126) is a viscoelastic material.

8. Clip holder in accordance with claim 7, **characterized in that** the viscoelastic material is a material which returns substantially completely to an original state following deformation.

9. Clip holder in accordance with claim 7 or 8, **characterized in that** the viscoelastic material has a relaxation time of at least approximately 0.5 seconds and/or of, at most, approximately 2 seconds, which elapses following deformation of the viscoelastic material up until at least partial restoration of an original state.

10. Clip holder in accordance with any one of claims 7 to 9, **characterized in that** the at least one holding device (122) comprises a viscoelastic material or is made of a viscoelastic material.

11. Clip holder in accordance with any one of claims 1 to 10, **characterized in that** the clip holder (100) comprises at least one clip (102).

12. Clip holder in accordance with claim 11, **characterized in that** the clip holder (100) comprises at least one coupling device (170) for coupling the at least one clip (102) to the at least one holding device (122).

13. Method of removing a medical or surgical clip (102) from a holding device (122) of a medical or surgical clip holder (100) for at least one medical or surgical clip (102), wherein at least two holding surface areas (124) of the holding device (122) are deflected relative to each other to release a connection with force locking and/or positive locking between the clip (102) and the holding device (122), wherein the at least one holding device (122) comprises at least one restoring device (128) for moving the at least two holding surface areas (124) relative to each other into a force-neutral position in which the at least one restoring device (128) holds the at least two holding surface areas (124) in a predefined position relative to each other following deflection of the at least two holding surface areas (124) relative to each other from an initial position into a deflected position, **characterized in that** the at least one holding device (122) comprises at least one damping device (126) counteracting the at least one restoring device (128) for damping a movement of the at least two holding surface areas (124) relative to each other from the deflected position back in the direction of the initial position, and **in that** the clip (102) is removed from the holding device (122) before the at least two holding surface areas (124) return to an initial position existing before deflection.

14. Method in accordance with claim 13, **characterized in that** a frictional effect between at least one holding surface area (124) of the holding device (122) and the clip (102), when removing the clip (102), is reduced by at least approximately 50 % by the deflection of the at least two holding surface areas (124) relative to each other to release the connection with force locking and/or frictional locking between the holding device (122) and the clip (102).

15. Use of a clip holder (100) in accordance with any one of claims 1 to 12 for performing a method in accordance with claim 13 or 14.

## Revendications

1. Système médical ou chirurgical de support de clips (100) comprenant au moins un dispositif de maintien (122) pour maintenir au moins un clip médical ou chirurgical (102), lequel dispositif de maintien (122) comprend au moins deux zones de surface de maintien (124) mobiles relativement l'une par rapport à l'autre pour le maintien par adhérence et/ou par complémentarité de formes dudit au moins un clip (102), ledit au moins un dispositif de maintien (122) comprenant au moins un dispositif de rappel (128) pour déplacer lesdites au moins deux zones de surface de maintien (124) l'une par rapport à l'autre dans une position neutre en force, dans laquelle ledit au moins un dispositif de rappel (128) maintient lesdites au moins deux zones de surface de maintien (124) dans une position relative prédéterminée l'une par rapport à l'autre, après une déviation desdites au moins deux zones de surface de maintien (124) l'une par rapport à l'autre, d'une position initiale à une position de déviation, **caractérisé en ce que** ledit au moins un dispositif de maintien (122) comprend au moins un dispositif d'amortissement (126), qui agit à l'encontre dudit au moins un dispositif de rappel (128), et qui est destiné à amortir un mouvement desdites au moins deux zones de surface de maintien (124) l'une par rapport à l'autre, en retour de la position de déviation vers la position initiale.

2. Système de support de clips selon la revendication 1, **caractérisé en ce que** le système de support de clips (100) comprend un corps de base (104), et **en ce qu'**au moins une zone de surface de maintien (124) est réalisée mobile par rapport au corps de base (104).

3. Système de support de clips selon l'une des revendications 1 ou 2, **caractérisé en ce que** ledit au moins un dispositif de maintien (122) comprend une surface d'appui (112) pour ledit au moins un clip (102).

4. Système de support de clips selon la revendication 3, **caractérisé en ce que** le système de support de clips (100) comprend au moins un élément d'appui (110) dont la surface forme au moins une partie de la surface d'appui (112) pour ledit au moins un clip (102).

5. Système de support de clips selon l'une des revendications 1 à 4, **caractérisé en ce que** ledit au moins un dispositif de rappel (128) et ledit au moins un dispositif d'amortissement (126) sont des pièces séparées dudit au moins un dispositif de maintien (122), ou **en ce que** ledit au moins un dispositif de rappel (128) est réalisé d'un seul tenant avec ledit au moins un dispositif d'amortissement (126).

6. Système de support de clips selon l'une des revendications 1 à 5, **caractérisé en ce que** ledit au moins un dispositif de rappel (128) et ledit au moins un dispositif d'amortissement (126) sont réalisés dans le même matériau.

7. Système de support de clips selon la revendication 6, **caractérisé en ce que** le matériau dudit au moins dispositif de rappel (128) et dudit au moins un dispositif d'amortissement (126) est un matériau viscoélastique.

8. Système de support de clips selon la revendication 7, **caractérisé en ce que** le matériau viscoélastique est un matériau qui, après une déformation, retourne sensiblement totalement dans un état initial.

9. Système de support de clips selon la revendication 7 ou la revendication 8, **caractérisé en ce que** le matériau viscoélastique présente un temps de relaxation, qui s'écoule après une déformation du matériau viscoélastique jusqu'à un rétablissement au moins partiel d'un état initial, d'une valeur d'au moins environ 0,5 seconde et/ou au plus d'environ 2 secondes.

10. Système de support de clips selon l'une des revendications 7 à 9, **caractérisé en ce que** ledit au moins un dispositif de maintien (122) englobe un matériau viscoélastique, ou est formé par un matériau viscoélastique.

11. Système de support de clips selon l'une des revendications 1 à 10, **caractérisé en ce que** le système de support de clips (100) comprend au moins un clip (102).

12. Système de support de clips selon la revendication 11, **caractérisé en ce que** le système de support de clips (100) comprend au moins un dispositif de couplage (170) destiné à assurer le couplage dudit au moins clip (102) avec ledit au moins un dispositif de maintien (122).

13. Procédé pour prélever un clip médical ou chirurgical (102) d'un dispositif de maintien (122) d'un système médical ou chirurgical de support de clips (100) pour au moins un clip médical ou chirurgical (102), d'après lequel au moins deux zones de surface de maintien (124) du dispositif de maintien (122) sont déviées relativement l'une par rapport à l'autre en vue d'interrompre une liaison par adhérence et/ou par complémentarité de formes entre le clip (102) et le dispositif de maintien (122), ledit au moins un dispositif de maintien (122) comprenant au moins un dispositif de rappel (128) pour déplacer lesdites au moins deux zones de surface de maintien (124) l'une par rapport à l'autre dans une position neutre en force, dans laquelle ledit au moins un dispositif de rappel (128) maintient lesdites au moins deux zones de surface de maintien (124) dans une position relative prédéterminée l'une par rapport à l'autre, après une déviation desdites au moins deux zones de surface de maintien (124) l'une par rapport à l'autre, d'une position initiale à une position de déviation, **caractérisé en ce que** ledit au moins un dispositif de maintien (122) comprend au moins un dispositif d'amortissement (126), qui agit à l'encontre dudit au moins un dispositif de rappel (128), et qui est destiné à amortir un mouvement desdites au moins deux zones de surface de maintien (124) l'une par rapport à l'autre, en retour de la position de déviation vers la position initiale, et **en ce que** le clip (102) est prélevé du dispositif de maintien (122), avant que lesdites au moins deux zones de surface de maintien (124) retournent dans une position initiale existant avant la déviation.

14. Procédé selon la revendication 13, **caractérisé en ce que** grâce à la déviation desdites au moins deux zones de surface de maintien (124) l'une par rapport l'autre, en vue d'interrompre la liaison par adhérence et/ou par complémentarité de formes entre le clip (102) et le dispositif de maintien (122), un effet de friction entre au moins une zone de surface de maintien (124) du dispositif de maintien (122) et le clip (102) est réduit d'au moins environ 50% lors du prélèvement du clip (102).

15. Utilisation du système de support de clips (100) selon l'une des revendications 1 à 12, pour la mise en oeuvre d'un procédé selon l'une des revendications 13 ou 14.
